# EUROPEAN PATENT SPECIFICATION

(11) **EP 4 628 940 B1**
(45) Date of publication and mention of the grant of the patent: **18.11.2026**
(21) Application number: 25164297.1
(22) Date of filing: 18.03.2025
(51) Int. Cl.: G01T 1/167, G01T 1/178, G01T 1/30, G01T 1/36

(54) **RADIONUCLIDE IMPURITY FRACTION ALGORITHM AND METHODS OF IMPLEMENTATION**
ALGORITHMUS FÜR RADIONUKLIDVERUNREINIGUNGSFRAKTION UND IMPLEMENTIERUNGSVERFAHREN
ALGORITHME DE FRACTION D'IMPURETÉS DE RADIONUCLÉIDES ET PROCÉDÉS DE MISE EN UVRE

(30) Priority: 19.03.2024 US 202463567248 P; 31.12.2024 US 202419007085
(43) Date of publication of application: 08.10.2025
(73) Proprietor: Mirion Technologies, Inc., Atlanta Georgia 30318 (US)
(72) Inventor: PERSSON, Henrik Ingvar, Guilford, Connecticut, 06437 (US); PHILLIPS, Kara Elizabeth, Brandford, Connecticut, 06437 (US)
(74) Representative: LLR

(56) References cited:
- CAFFARI Y ET AL: "Activity measurements of ^1^8F and ^9^0Y with commercial radionuclide calibrators for nuclear medicine in Switzerland", APPLIED RADIATION AND ISOTOPES, ELSEVIER, OXFORD, GB, vol. 68, no. 7-8, July 2010 (2010-07-01), pages 1388 - 1391, XP027053686, ISSN: 0969-8043, [retrieved on 20091111]
- "Nuclear instrumentation - Measurement of activity or emission rate of gamma-ray emitting radionuclides - Calibration and use of germanium-based spectrometers", 4 June 2021 (2021-06-04), pages 1 - 98, XP082028022, Retrieved from the Internet <URL:https://api.iec.ch/harmonized/publications/download/1063812> [retrieved on 20210604]
- JUDGE S M ET AL: "Traceability for nuclear medicine: the status of primary radioactivity standards", METROLOGIA, INSTITUTE OF PHYSICS PUBLISHING, BRISTOL, GB, vol. 60, no. 1, 20 December 2022 (2022-12-20), XP020440557, ISSN: 0026-1394, [retrieved on 20221220], DOI: 10.1088/1681-7575/ACA67A
- KORUN M ET AL: "Calculation of the best estimates for measurements of radioactive substances when the presence of the analyte is not assured", ACCREDITATION AND QUALITY ASSURANCE, SPRINGER BERLIN HEIDELBERG, BERLIN/HEIDELBERG, vol. 21, no. 3, 22 March 2016 (2016-03-22), pages 191 - 195, XP035811591, ISSN: 0949-1775, [retrieved on 20160322], DOI: 10.1007/S00769-016-1198-8
- ZORKO BENJAMIN ET AL: "Systematic influences of gamma-ray spectrometry data near the decision threshold for radioactivity measurements in the environment", JOURNAL OF ENVIRONMENTAL RADIOACTIVITY., vol. 158-159, July 2016 (2016-07-01), GB, pages 119 - 128, XP093303706, ISSN: 0265-931X, DOI: 10.1016/j.jenvrad.2016.04.009
- SIMON JÁN ET AL: "Basic Statistical Properties of the Knot Efficiency", SYMMETRY, vol. 14, no. 9, 15 September 2022 (2022-09-15), pages 1926, XP093303256, ISSN: 2073-8994, DOI: 10.3390/sym14091926

## Description

### TECHNICAL FIELD

This disclosure generally relates to detection of radionuclides, and more specifically to a new technique of determining an upper limit with a specified confidence of the ratio of measurements of the activities and their uncertainties of two radionuclides.

### BACKGROUND

When radionuclides are produced to be used as pharmaceuticals there is a possibility that the sample will contain other radionuclides that are not desirable. For example, during many production processes other radionuclides or radioisotopes are often produced together with the desired radionuclide. The radionuclides that are used as pharmaceuticals are usually called primary radionuclides while the undesired radionuclides are typically called impurities. Regulations say that the activity of the impurities are required to be less than a fraction of activity of the primary radionuclide.

Some impurities can be chemically separated from the desired or primary radionuclides in a sample. The activity of the impurities that remains after the separation can be expressed as a percentage or fraction of the total activity of the sample, sometimes called the percent impurity. However, in use cases where all of the impurities are able to be chemically separated or little to no impurities are produced, the activity of the sample is entirely or almost entirely made of the activity of the primary radioisotope. In those cases, users may report the radionuclide impurity as a percentage of the primary radionuclide/radioisotope activity rather than as a percentage of total sample activity.

Literature search and interactions with users have revealed that the activity of the impurity divided by the activity of the primary radionuclide has been used when the impurity has been identified. However, this approach does not take uncertainties in these activities into account, and these can be large.

CAFFARI Y ET AL: "Activity measurements of 18F and 90Y with commercial radionuclide calibrators for nuclear medicine in Switzerland", APPLIED RADIATION AND ISOTOPES, ELSEVIER, OXFORD, GB, vol. 68, no. 7-8, 1 July 2010 (2010-07-01), pages 1388-1391, discloses radionuclide activity measurements and calculation of uncertainties of activities of primary radionuclides.

### SUMMARY

When the radionuclide has not been identified, the most common approach by users currently is to use the Minimum Detectable Activity (MDA) [1] for the impurity and divide it with the activity of the primary radionuclide. The definition of the MDA does not support the use of the quantity in this way. The disclosed principles propose that a better and more defendable way is to calculate the activity and uncertainty of the impurity and primary radionuclide, and calculate the probability density of the ratio of the two normal probability density functions. From the probability density of the ratio, one can define an upper limit that the ratio can have with a certain confidence, and this number can be compared to the regulatory limit of the ratio of the activities in order to determine compliance with that regulatory limit.

This disclosure provides novel processes and methods of use for determining compliance with a maximum regulatory limit of an impurity radionuclide as compared to a primary radionuclide. The disclosed processes may be employed to calculate the upper limit, optionally a lower limit, and a probability that the ratio is below a user-defined limit (which is expected to be defined as the regulatory limit) based on the probability density of the ratio of the radionuclide activities and the uncertainties. The upper limit value is called the "maximum potential percent impurity" throughout this disclosure, which is of most relevancy to end users. The performance of the disclosed principles is demonstrated for an example of Monte Carlo simulations drawn from two normal probability density functions, and for an example of Monte Carlo simulations of gamma spectrometry measurements of Yb-169 and Yb-175 impurities in a Lu-177 sample. The disclosed principles and exemplary methods of practical implementation in general are applicable to any radionuclide measurement that measures the activity and uncertainty of two radionuclides. The expected use case is in context of gamma spectrometry.

Thus, it is provided according to the invention a method of determining an upper limit with a specified confidence of the ratio of measurements of the activities and their uncertainties of two radionuclides in a sample, the method comprising:
measuring activity of a primary radionuclide and activity of an impurity radionuclide;
calculating an uncertainty of the measured activity of the primary radionuclide and an uncertainty of the measured activity of the impurity radionuclide;
determining a probability density of activity of the primary and impurity radionuclides based on the measured activity and calculated uncertainty of each of the primary and impurity radionuclides;
establishing a probability density of the activity ratio of the impurity radionuclide to the primary radionuclide based on the determined probability density of the primary and impurity radionuclide, respectively;
determining a normalization factor (N) to offset when the determined ratio probability density of the activity ratio of the impurity and primary radionuclides is non-zero for negative values;
calculating an upper limit to a specified confidence of the activity ratio of the impurity and primary radionuclides given the measured activities and uncertainties and a-priori knowledge that the activity ratio cannot be negative; and
indicating that the activity ratio of an impurity radionuclide to the primary radionuclide is below a predetermined limit if the calculated upper limit is equal to or below the predetermined limit.

According to an embodiment of the invention, the measuring activity does not require a test that the measured activity of the impurity radionuclide is present.

According to an embodiment of the invention, the measuring activity comprises calculating a signal of interest from a background signal subtracted from a gross signal, wherein the background signal is estimated from previous measurements or from information from the current measurement, and wherein uncertainty of the calculated signal of interest is a positive value.

According to an embodiment of the invention, measuring activity for the impurity radionuclide comprises measuring radiation emitted from the impurity radionuclide at different energies by spectrometry, wherein calculating the upper limit of the activity ratio for the impurity radionuclide to the primary radionuclide comprises calculating multiple upper limits for respective different energies, and wherein the upper limit for the impurity radionuclide comprises the lowest of the multiple upper limits of the activity ratios.

Preferably, the minimum possible activity ratio for the impurity radionuclide comprises the lowest of the minimum possible activity ratios for all the activities of the radionuclide.

According to an embodiment of the invention, determining the normalization factor comprises calculating the integral of the ratio probability density from negative infinity to 0.

Preferably, the normalization factor is based on a Cumulative Distribution Function (CDF) responsive to determining the integral of the probability density function of the activity ratio from negative infinity to 0.

According to an embodiment of the invention, the probability density of the activity ratio is zero for negative activity ratios: ${f}_{Z} \left(z\left|Z\geq 0\right.\right) = \left\{\begin{matrix}1 \left(z\geq 0\right) \\ 0 \left(z<0\right)\end{matrix}\right.$

According to an embodiment of the invention, the normalization factor for the ratio probability density of the primary and impurity radionuclides comprises: $N = \frac{1}{{\int }_{0}^{z = ∞} {f}_{Z} dz} = \frac{1}{1 - {\int }_{z = - ∞}^{0} {f}_{Z} dz} = \frac{1}{1 - F \left(0\right)}$

According to an embodiment of the invention, calculating the maximum possible activity ratio comprises calculating zᵤ for which the normalized Probability Density Function (PDF) integral, as normalized by the normalization factor, is equal to 1 - α, where α is the significance level, as defined by: ${F}_{Z} \left({z}_{u}\left|{μ}_{x},{σ}_{{u}_{x}},{μ}_{y},{σ}_{{μ}_{y}},Z\geq 0\right.\right) = \frac{{F}_{Z} \left({z}_{u}\right) - {F}_{Z} \left(0\right)}{1 - {F}_{Z} \left(0\right)} = 1 - α$

According to an embodiment of the invention, calculating the minimum possible activity ratio comprises calculating *z*ₗ for which the normalized Probability Density Function (PDF) integral, as normalized by the normalization factor, is equal to α, where α is the significance level, as defined by: ${F}_{Z} \left({z}_{l}\left|{μ}_{x},{σ}_{{u}_{x}},{μ}_{y},{σ}_{{μ}_{y}},Z\geq 0\right.\right) = \frac{{F}_{Z} \left({z}_{l}\right) - {F}_{Z} \left(0\right)}{1 - {F}_{Z} \left(0\right)} = α$

According to an embodiment of the invention, calculating a probability that the true value of the ratio is below a specific limit *zᵥ* is calculated by: $\frac{{F}_{Z} \left({z}_{v}\right) - {F}_{Z} \left(0\right)}{1 - {F}_{Z} \left(0\right)}$

According to an embodiment of the invention, the measuring activity comprises measuring activity using gamma spectrometry.

According to an embodiment of the invention, the method further comprises calculating a lower limit to a specified confidence of the activity ratio of the impurity and primary radionuclides given the measured activities and uncertainties and the a-priori knowledge that the activity ratio cannot be negative.

According to an embodiment of the invention, determining a probability density of activity of the primary and impurity radionuclides is further based on the activity ratio of the impurity radionuclide as a percentage of the total activity in the sample.

According to an embodiment of the invention, determining a probability density of activity of the primary and impurity radionuclides is further based on the activity ratio of the impurity radionuclide as a percentage of the activity of the primary radionuclide in the sample.

### BRIEF DESCRIPTION OF THE FIGURES

The invention will be described on the basis of the following non-limitative embodiments making reference to the annexed drawings which comprise:
[Fig. 1A] and [Fig. 1B] respectively illustrate a plot of probability densities for the primary radionuclide activity and a plot of probability densities for the impurity radionuclide activity,
[Fig. 2] illustrates a plot of the probability density for the ratio probability density of the activities of the primary and impurity radionuclides,
[Fig. 3A] and [Fig. 3B] respectively illustrate a plot of the probability density of the activities of the primary radionuclides and a plot of probability density of the activities of the impurity radionuclides,
[Fig. 4] illustrates a plot of the ratios of the two sets of random numbers for the probability densities shown in [Fig. 3A] and [Fig. 3B],
[Fig. 5] illustrates a plot showing the maximum possible impurity fraction, the minimum possible impurity fraction, the MDA divided by primary activity, the impurity limit, and impurity activity divided by the primary activity, and the probability that the impurity fraction is below the limit for Yb-169 and a sample without Yb-169 impurity
[Fig. 6] illustrates a plot substantially similar to the plot of [Fig. 5], but for Yb-175 with a fractional impurity limit of 1e-3,
[Fig. 7] illustrates a plot substantially similar to the plot of [Fig. 5], but for the simulation containing an activity of Yb-169 at the limit, and
[Fig. 8] illustrates a plot substantially similar to the plot of [Fig. 7], but for Yb-175.

### DESCRIPTION OF EXEMPLARY EMBODIMENTS

Assume that the activity and uncertainty of two radionuclides X and Y have been measured, that the probability density for the true activities for the radionuclides are normally distributed with probability densities *N_{X}*(*µ_{X}, σ_{X}*) and *N_{Y}*(*µ_{Y}, σ_{Y}*), and that they are not correlated. The ratio *Z* of the probability densities of the activities is then: $Z = \frac{X}{Y} = \frac{{N}_{X} \left({μ}_{X}, {σ}_{X}\right)}{{N}_{Y} \left({μ}_{Y}, {σ}_{Y}\right)}$ The exact expression of probability density ${f}_{Z}^{∗}$ for the activity ratio $Z = \frac{X}{Y}$ given the measurements *µₓ* and *µ_{y}* with uncertainties *σ_{µₓ}* and *σ_{µ_{y}}* is: ${f}_{Z}^{∗} \left(z\left|{μ}_{x}, {σ}_{{u}_{x}}, {μ}_{y}, {σ}_{{μ}_{y}}\right.\right) = \frac{b \left(z\right) d \left(z\right)}{{a}^{3} \left(z\right)} \frac{1}{\sqrt{2 π} {σ}_{x} {σ}_{y}} \left(Φ\left(\frac{b \left(z\right)}{a \left(z\right)}\right)-Φ\left(\frac{- b \left(z\right)}{a \left(z\right)}\right)\right) + \frac{1}{{a}^{2} \left(z\right) {πσ}_{x} {σ}_{y}} {e}^{- \frac{c}{2}}$ where: $a \left(z\right) = \sqrt{\frac{1}{{σ}_{x}^{2}} {z}^{2} + \frac{1}{{σ}_{y}^{2}}}$ $b \left(z\right) = \frac{{μ}_{x}}{{σ}_{x}^{2}} z + \frac{{μ}_{y}}{{σ}_{y}^{2}}$ $c \left(z\right) = \frac{{μ}_{x}^{2}}{{σ}_{x}^{2}} + \frac{{μ}_{y}^{2}}{{σ}_{y}^{2}}$ $d \left(z\right) = {e}^{\frac{{b}^{2} \left(z\right) - {ca}^{2} \left(z\right)}{2 {a}^{2} \left(z\right)}}$ and Φ is the cumulative distribution function of the normal probability density function. For the special case where the relative uncertainty of the measured activity Y is small, i.e., $\frac{{σ}_{{μ}_{y}}}{{μ}_{y}}$ is small, the probability density function can be approximated to be normally distributed. This is not necessarily the case for radionuclidic purity measurements.

However, the above expression is computationally expensive to calculate and thus the cumulative distribution needs to be computed using numerical integration. Simon and Ftorek [2], showed that an approximation exists that gives indistinguishable results, and it is valid for all practical applications. The approximation ${f}_{Z}^{†}$, also referred to as the "solid approximation", is: ${f}_{Z}^{†} \left(z\left|{μ}_{x}, {σ}_{{u}_{x}}, {μ}_{y}, {σ}_{{μ}_{y}}\right.\right) = \frac{1}{\sqrt{π}} \frac{p}{erf \left(q\right)} \frac{1}{r} \frac{1 + \frac{{p}^{2}}{{q}^{2}} \frac{z}{r}}{{\left(1+\frac{{p}^{2}}{{q}^{2}}\frac{{z}^{2}}{{r}^{2}}\right)}^{\frac{3}{2}}} {e}^{- \frac{{p}^{2} {\left(\frac{z}{r}-1\right)}^{2}}{1 + \frac{{p}^{2} {z}^{2}}{{q}^{2} {r}^{2}}}}$ where *erf* is the error function, $p = \frac{{μ}_{X}}{\sqrt{2} {σ}_{X}} ,$ $q = \frac{{μ}_{Y}}{\sqrt{2} {σ}_{Y}} ,$ $r = \frac{{μ}_{X}}{{μ}_{Y}} .$ They also show that the solid approximation has the following Cumulative Distribution Function (CDF) ${F}_{Z}^{†} \left({z}_{0}\right)$ (the probability that the true value is less than or equal to *z*₀): ${F}_{Z}^{†} \left({z}_{0}\left|{μ}_{x}, {σ}_{{u}_{x}}, {μ}_{y}, {σ}_{{μ}_{y}}\right.\right) = {f}_{Z}^{†} \left(z<{z}_{0}\left|{μ}_{x}, {σ}_{{u}_{x}}, {μ}_{y}, {σ}_{{μ}_{y}}\right.\right) = \frac{1}{2} \left(1+\frac{erf \left(\frac{p \left(\frac{{z}_{0}}{r}-1\right)}{\sqrt{1 + \frac{{p}^{2}}{{q}^{2}} \frac{{z}_{0}^{0}}{{r}^{2}}}}\right)}{erf \left(q\right)}\right) .$

The solid approximation is less computation intensive, and the Cumulative Distribution Function can be evaluated directly instead of using numerical integration, resulting in a much faster (i.e., easier; less computationally expensive) computation to compute. It is not possible to use the solid approximation if the mean of the activities of either of the radionuclides are 0. This can happen for radionuclide measurements if the count time is not long enough to register any counts. In this case, it is possible to revert to the exact solution. However, the fact remains that it is not possible to calculate the probability density if the uncertainty of the radionuclide activity is 0 for either of the radionuclides. All measurements should have an uncertainty that is larger than 0, but in radionuclide measurements this happens when there are no counts registered (for whatever reason) and the uncertainty is calculated from the square root of number of counts. In such cases, it is not possible to recover, and the Probability Density Function (PDF)(the probability that a variable will take a value exactly equal to that variable) or the CDF cannot be evaluated.

### Bayesian A Priori Knowledge of the PDF

It is known *a priori* that the radionuclide activities cannot be negative. This means that the activity ratio cannot be negative either. There is no additional *a priori* knowledge of the activity ratio. Adapting the formalism in ISO11929 [3,4] on including a non-negative *a priori* knowledge to this specific case by multiplying the probability density function by the *a priori* knowledge of non-negativity activity ratio: ${f}_{Z} \left(z\left|Z\geq 0\right.\right) = \left\{\begin{matrix}1 \left(z\geq 0\right) \\ 0 \left(z<0\right)\end{matrix}\right.$ to get a probability density function for the activity ratio Z, given the measurements *µₓ* and *µ_{y}* with uncertainties *σ_{µₓ}* and *σ_{µ_{y}}* with the prior knowledge that the activity ratio cannot be negative, that is: ${f}_{Z} \left(z\left|{μ}_{x},{σ}_{{u}_{x}},{μ}_{y},{σ}_{{μ}_{y}},Z\geq 0\right.\right) = {Nf}_{Z} \left(z\left|{μ}_{x}, {σ}_{{u}_{x}}, {μ}_{y}, {σ}_{{μ}_{y}}\right.\right) {f}_{Z} \left(z\left|Z\geq 0\right.\right)$ where N is a normalization constant to ensure that the integral from 0 to infinity is 1.0. The normalization constant is: $N = \frac{1}{{\int }_{0}^{z = ∞} {f}_{Z} dz} = \frac{1}{1 - {\int }_{z = - ∞}^{0} {f}_{Z} dz} = \frac{1}{1 - F \left(0\right)}$

Finally, we can write the full expression of the probability density function as: ${f}_{Z} \left(z\left|{μ}_{x},{μ}_{y},,{μ}_{y},{σ}_{{μ}_{y}}\right.,Z\geq 0\right)$ and the cumulative distribution function as: ${F}_{Z} \left({z}_{0}\left|{μ}_{x}, {σ}_{{u}_{x}}, {μ}_{y}, {σ}_{{μ}_{y}}\right.,Z\geq 0\right)$ including the *a priori* knowledge of non-negative as: ${F}_{Z} \left(z\left|{μ}_{x},{σ}_{{u}_{x}},{μ}_{y},{σ}_{{μ}_{y}},Z\geq 0\right.\right) = \left\{\begin{matrix}\frac{{f}_{Z}}{1 - {F}_{Z} \left(0\right)} & z \geq 0 \\ 0 & z < 0\end{matrix},\right.$ ${F}_{z} \left({z}_{0}\left|{μ}_{x},{σ}_{{u}_{x}},{μ}_{y},{σ}_{{μ}_{y}},Z\geq 0\right.\right) = \left\{\begin{matrix}\frac{{F}_{Z} \left({z}_{0}\right) - {F}_{Z} \left(0\right)}{1 - {F}_{Z} \left(0\right)} & {z}_{0} \geq 0 \\ 0 & {z}_{0} < 0\end{matrix}\right.$ The expressions above can either be evaluated using the exact probability density ${f}_{Z}^{∗} \left(z\right)$ and numerical integration to calculate the cumulative distribution function or using the solid approximation ${f}_{Z}^{†}$ and the cumulative distribution function ${F}_{Z}^{†}$. The solid approximation is much easier to calculate, and thus, in accordance with the disclosed principles, it should be used whenever possible.

### Calculation of Activities from Radionuclides

The signal of the radionuclide being measured is often calculated from a gross signal G, which consists of signal of interest S and a background signal B: $S = G - B$ The background signal is then estimated from previous measurements or from other information from the same measurement. Both the measurement of the gross signal and the estimation of the background signal have an uncertainty associated with them.

When the signal is small compared to the background signal, it is possible that the measurement of the gross signal is smaller than the estimation of the background signal and the value of the signal then becomes negative. The uncertainty of the signal should be consistent with 0 or a positive value of the signal. For example, in gamma spectrometry the background signal can consist of Compton continuum, environmental background, and interfering signals from other radionuclides in the sample.

The measured signal is converted to activity using a conversion factor w that also has an uncertainty. The uncertainty of the activity is the combination of the uncertainty in the signal and the uncertainty of the conversion factor w. When using this method, the activity should be calculated without using any test if the signal is statistically significant or not.

### Calculation of the Upper and Lower limit of the Activity Ratio

Now, calculation of the upper and lower limit of the activity ratio with a specified confidence can be done with the following steps:
1. Calculate the activity and uncertainty of the impurity radionuclide using the standard algorithms, for example, using gamma spectrometry.
2. Calculate the activity and uncertainty of the primary radionuclide using standard algorithms, for example, using gamma spectrometry or using the activity and uncertainty measured with a different instrument.
3. Calculate the integral of the probability density from 0 to infinity. This can be done by calculating 1.0 - *F_{Z}*(0)*.* Use this value as the normalization factor *N*.
4. Calculate zᵤ for which the normalized PDF integral is equal to 1 - *α*, where *α* is the significance level. Calculate *F_{Z}* (*z*ᵤ|*µₓ, σ_{uₓ}, µ_{y}, σ_{u_{y}}, Z* ≥ 0) = 1 - α. Using the CDF this can be calculated as $\frac{{F}_{Z} \left({z}_{u}\right) - {F}_{Z} \left(0\right)}{1 - {F}_{Z} \left(0\right)}$*.* The value of zᵤ can be found by iteration, for example, using the bisection method. This is the maximum possible percent impurity ratio for confidence 1 - α.
5. Calculate *zₗ* for which the normalized PDF integral is equal to the confidence factor *α*. Calculate *F_{Z}* (*z*ₗ|*µₓ*, *σ_{uₓ}, µ_{y}, σ_{µ_{y}}, Z* ≥ 0) *= α*. Using the CDF, this can be calculated as $\frac{{F}_{Z} \left({z}_{l}\right) - {F}_{Z} \left(0\right)}{1 - {F}_{Z} \left(0\right)}$. The value of zₗ can be found by iteration, for example, using the bisection method. This is the minimum possible ratio for confidence *α*.
6. The probability that the true value of the ratio is below a specific value zᵥ can be calculated by evaluating $\frac{{F}_{Z} \left({z}_{v}\right) - {F}_{Z} \left(0\right)}{1 - {F}_{Z} \left(0\right)}$.

Many radionuclides emit radiation with several different energies that can be recorded and separated in a spectrum. This means that it is possible to calculate more than one value of the maximum possible percent impurity for a radionuclide. These values may be vastly different for radiation emitted at different energies, because of different detector response and emission probability. Gamma spectrometry is one example where this is possible. The maximum possible ratio for the radionuclide is the lowest of the maximum possible ratios for all the emissions for the radionuclide. The minimum possible ratio for the radionuclide is the lowest of the minimum possible ratios for all the emissions of the radionuclide. The probability that the activity of the radionuclide is below the limit is the largest probability calculated for any emission of the radionuclide.

Additionally, when determining the probability density of activity of the primary and impurity radionuclides, the activity ratio of the impurity radionuclide as a percentage of the total activity in a sample is typically used, for example, when the impurities remain in the sample even after chemical separation of some impurities is performed. However, in cases where the activity of the sample is almost entirely made of the activity of the primary radionuclide, then when determining the probability density of activity of the primary and impurity radionuclides, the activity ratio of the impurity radionuclide as a percentage of the activity of the primary radionuclide in the sample may be used.

### Testing of Implementation of the Disclosed Principles

The disclosed principles have been tested in two ways. The first was a comparison to simulations of ratio of two normally distributed random variables. The second is applied to simulated measurements of Lu-177.

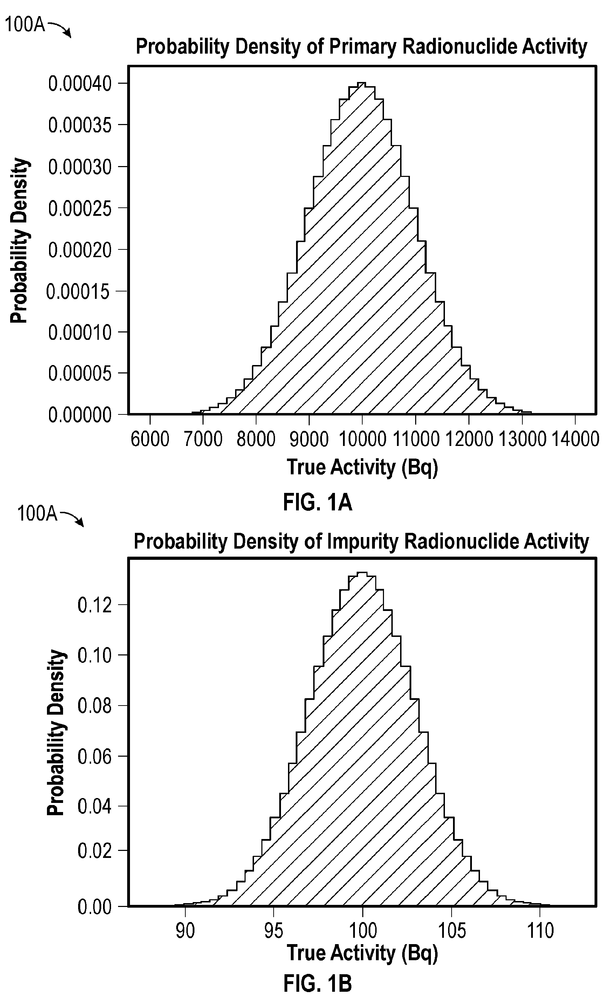

What can be drawn are two sets of normally distributed random variables and the ratio of these taken. If any of the two random variables are negative, the pair is discarded because of the *a priori* knowledge that the radionuclide activities cannot be negative. For example, using a mean of 10,000 and standard deviation of 1,000 for the set Y (the primary radionuclide), and mean 100 and a standard deviation of 3 for the set X (the impurity radionuclide), one can draw 20 million random numbers from each probability density function and calculate the ratios of them. **FIG. 1A** illustrates a plot 100A of the probability densities for the primary (Y) radionuclide activity. **FIG. 1B** illustrates a plot 100B of the probability densities for the impurity (X) radionuclide activity. As expected, the densities look like the normal densities.

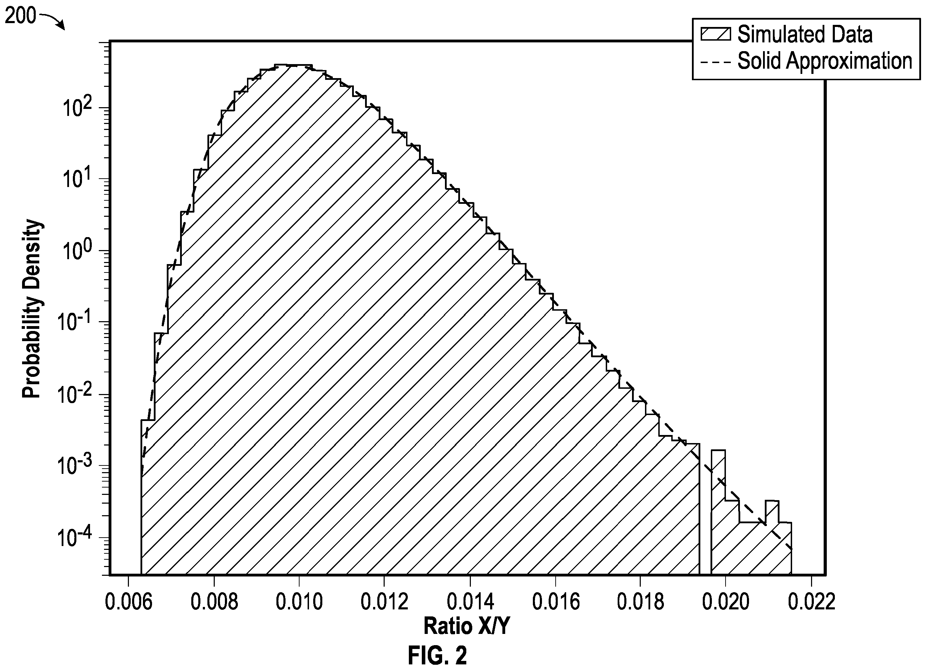

**FIG. 2** illustrates a plot 200 of the probability density for the ratio of X/Y (the ratio probability density of the activities of the primary and impurity radionuclides) for the simulated data plotted together with the calculation from the solid approximation of the probability density function. As shown in the plot 200, the distribution of the probability density is not symmetric, and it does not follow the normal (conventional) probability density function. As illustrated, the solid approximation of the probability density function provided by the disclosed principles reproduces the simulated data very well. The simulated data has a larger tail to higher ratios, which means that using a normal density when calculating the maximum possible ratio would yield a value that is lower than the true maximum ratio.

Employing the disclosed principles, one can now calculate the 99th percentile of the simulated data, which represents the maximum possible ratio with α equal to 1% or with 99% confidence that the true ratio is below this value and the same value using the disclosed principles and equations described above. One can also compare it to using a normal probability density function for the ratio and calculate the maximum possible ratio with 99% confidence. The results are summarized in **Table 1.**

**Table 1: Calculation of the maximum possible ratio at 99% confidence for simulated data, the solid approximation provided by the disclosed principles, and normal approximation provided by conventional techniques.**

| **Method** | **Maximum Possible Ratio** | **Relative Difference from Simulation** |
|---|---|---|
| Simulation | 1.313e-2 | N/A |
| Solid approximation | 1.313e-2 | -0.0011 % |
| Normal approximation | 1.243e-2 | -5.4 % |

The solid approximation provided by the disclosed principles reproduces the simulation almost exactly, while using a normal/conventional probability density approximation calculates a value over 5% below the simulated data. It is unlikely to have a 10% relative uncertainty of the activity of the primary radionuclide, but by using the more accurate method as disclosed herein, a user would not have to worry about how large the uncertainty in the activity primary radionuclide can be before the conventional method gives an inaccurate answer.

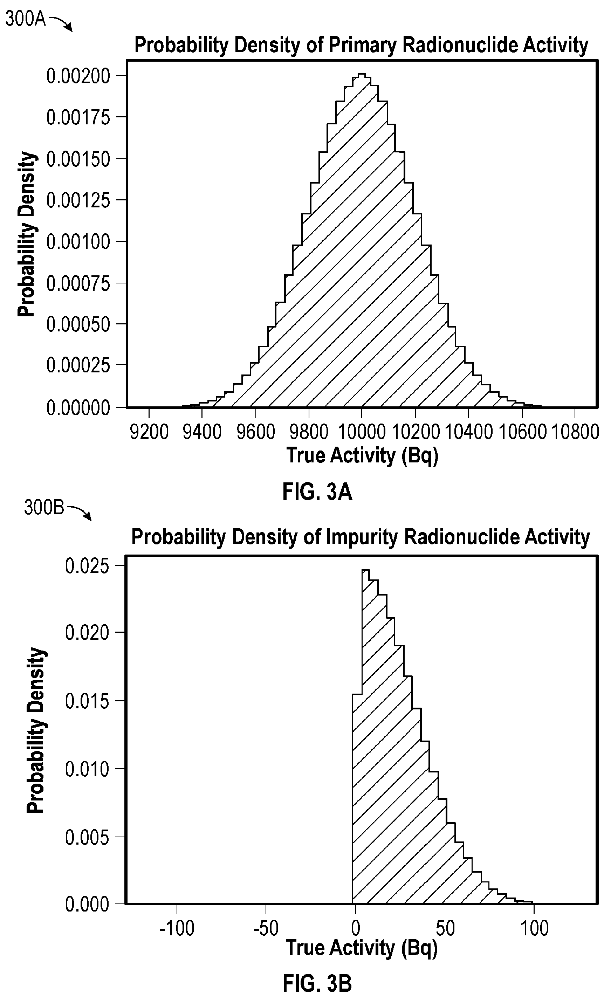

In the second example, a mean of 10,000 with a standard deviation of 200 for the primary radionuclide (Y) is used and a mean of 3 with a standard deviation of 30 for the impurity radionuclide X is used. One can again draw 20,000,000 random numbers from the two probability density functions and because the negative activities need to be rejected, there will be about 10.8 million ratios of X to Y. **FIG. 3A** illustrates a plot 300A of the probability density of the activities of the primary (Y) radionuclides, and **FIG. 3B** illustrates a plot 300B of the probability density of the activities of the impurity (X) radionuclides. The activity probability density of the primary radionuclide (Y) is following the normal probability density function, while the activity probability density of the impurity radionuclide (X) is a truncated normal probability density function (i.e., no negative values).

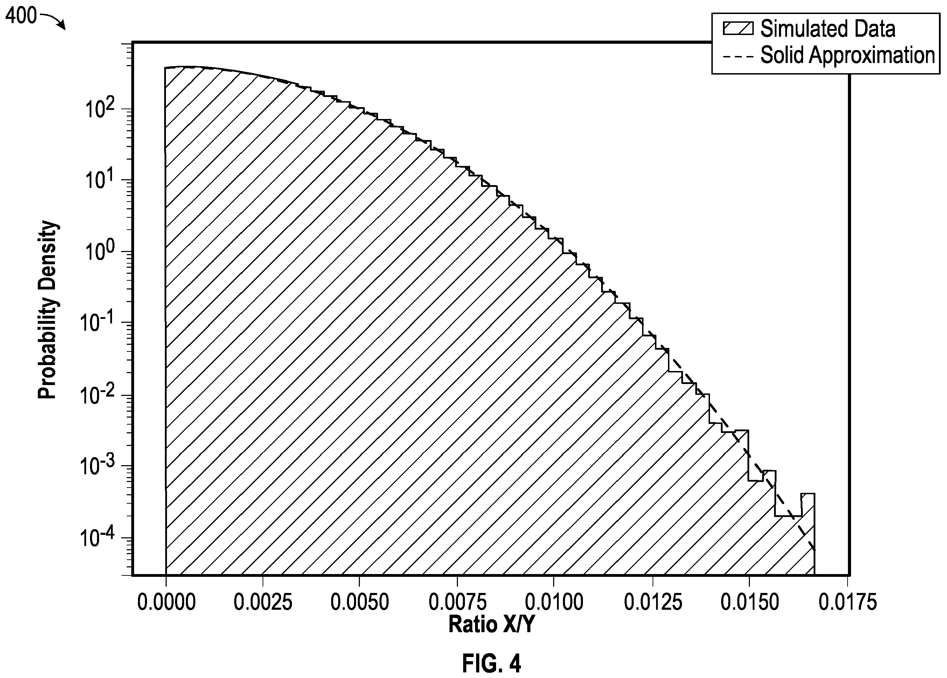

**FIG.** 4 illustrates a plot 400 of the ratios of the two sets of random numbers for the probability densities shown in FIGS. 3A and 3B. The normalized solid approximation is also shown in this same figure. As illustrated, the agreement between the simulated data and the normalized solid approximation provided by the disclosed principles is excellent.

The 99th percentile of the simulated data, the maximum possible ratio at the 99% confidence calculated using the solid approximation using the *a priori* knowledge and the maximum possible ratio using a normal approximation without the *a priori* knowledge is shown in **Table 2.**

**Table 2: Calculation of the maximum possible ratio at 99% confidence for simulated data, the solid approximation provided by the disclosed principles, and normal approximation provided by conventional techniques.**

| **Method** | **Maximum Possible Ratio** | **Relative Difference From Simulation** |
|---|---|---|
| Simulation | 7.96e-03 | N/A |
| Solid approximation | 7.96e-03 | -0.031 % |
| Normal approximation | 7.28e-03 | -8.6 % |

The solid approximation reproduces the simulated data almost exactly, while the normal approximation without using the *a priori* knowledge results in an underestimation of the maximum possible ratio by over 8%. This shows that using the *a priori* knowledge as disclosed herein is crucial to be able to calculate the maximum possible ratio accurately in cases with very large relative uncertainty in the activity of the impurity radionuclide. Such situations are the common case, thus making the disclosed approach even more crucial to implement in the typical use cases.

In the next test, the performance of the disclosed principles on spectral data generated using MCNP-CP calculations was tested. The simulation was a Lu-177 sample with no impurity and at the limit. The impurities were Yb-169 and Yb-175 with 1e-4 and 1e-3 limits, respectively. The geometry used was a liquid scintillation vial at 10 cm from a 10% relative efficiency High Purity Germanium (HPGe) detector. 400 Monte Carlo radiation transport simulations of 1e7 decays each were performed and the simulations were summed and converted to a format compatible with gamma spectrometry software.

The first file contained 1e7 decays, the second file contained the initial 1e7 decays and an additional 1e7 decays and so on. This resulted in 400 spectra with increasing statistics to be able to track how the algorithm performs over time. The measurement time was set to 10 seconds for the first spectrum and 20 seconds for the second and so on. This was done for the 2 levels of impurities. All spectra were analyzed using standard gamma spectrometry algorithms to generate radionuclide activities and uncertainties. The library peak search algorithm was used to force the gamma spectrometry algorithms to find peaks at the library energies. For Yb-169 only the 307 keV emissions were used for the nuclide maximum possible ratio, all other emissions were flagged as "do not use for weighted mean calculation". For Yb-175 the 282 and 396 keV lines were used for calculating the nuclide maximum possible ratio.

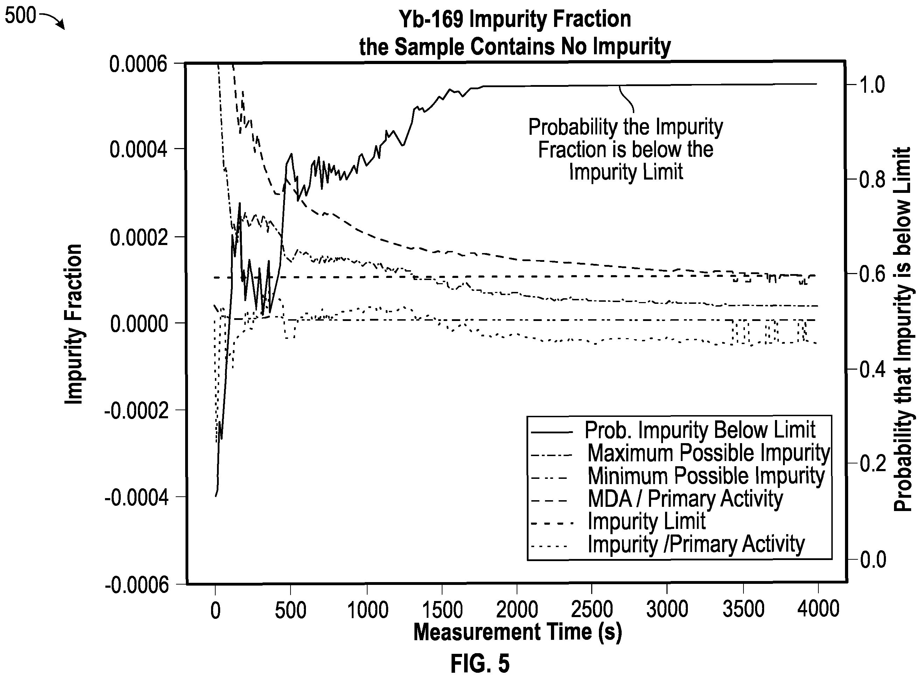

**FIG. 5** illustrates a plot 500 showing the maximum possible impurity fraction, the minimum possible impurity fraction, the MDA divided by primary activity, the impurity limit, and impurity activity divided by the primary activity, and the probability that the impurity fraction is below the limit for Yb-169 and a sample without Yb-169 impurity. Note that the probability is plotted using the scale on the right-hand side of the plot.

Looking at the maximum possible impurity curve, it behaves as expected. The trend is that it decreases when the time and statistics increase, and after a certain time it goes below the impurity limit at which point it is possible with 95% certainty to say that the impurity fraction is below the limit. It is also important to note that the maximum possible impurity fraction is always below the MDA / primary activity. This means that it is possible to draw the conclusion that the impurity fraction is below the desired limit using this method with less than half the measurement time compared to using the MDA / primary activity. The probability that the impurity fraction is below the limit behaves as expected. Initially, there is a lot of fluctuation around 0.5 seconds. However, as time and statistics increase, the probability increases steadily and stabilizes at 1.0, which means that the equations and calculations, and overall methods, performed in accordance with the disclosed principles are completely certain that the impurity fraction is below the limit.

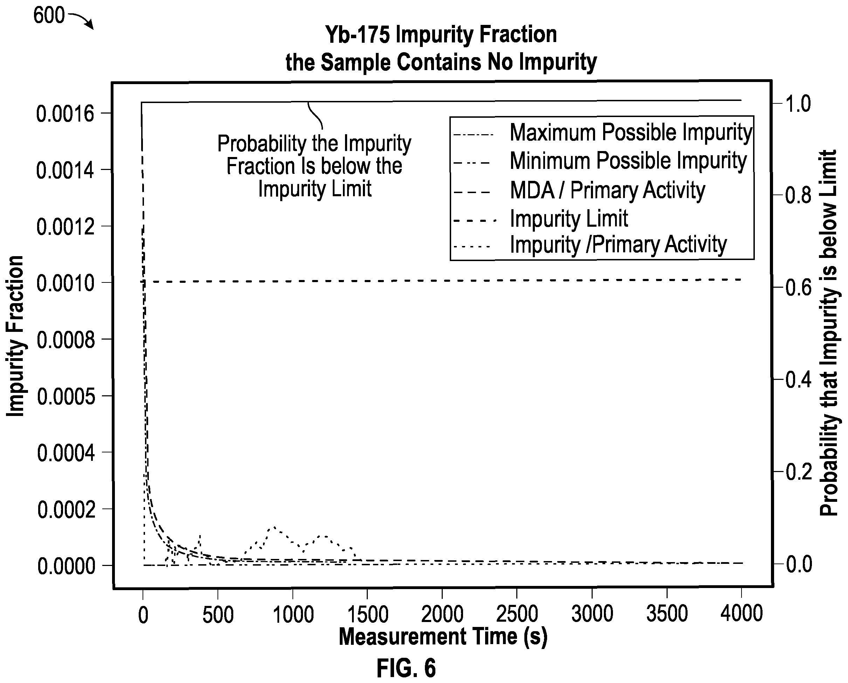

**FIG. 6** illustrates a plot 600 substantially similar to the plot 500 of FIG. 5, but for Yb-175 with a fractional impurity limit of 1e-3. The simulated sample contains no activity of Yb-175. This plot 600 illustrates the maximum possible impurity fraction, the minimum possible impurity fraction, the MDA divided by primary activity, the impurity limit, impurity activity divided by the primary activity, and the probability that the impurity fraction is below the limit for Yb-175 and a sample without Yb-175 impurity. Note again that the probability is plotted using the scale on the right-hand side of the plot.

One of the emissions from Yb-175 has an energy higher than the highest energy of Lu-177, which means that there is a very small continuum in this case, and very small uncertainty of the Yb-175 activity. The maximum possible impurity fraction goes below the limit and the probability of being below the limit goes to 1.0 almost immediately. The MDA / primary activity has the same behavior. This shows that for the cases where it is very easy to reach the impurity fraction limit, there is very little difference between using the maximum possible impurity fraction algorithm and using the MDA / primary activity.

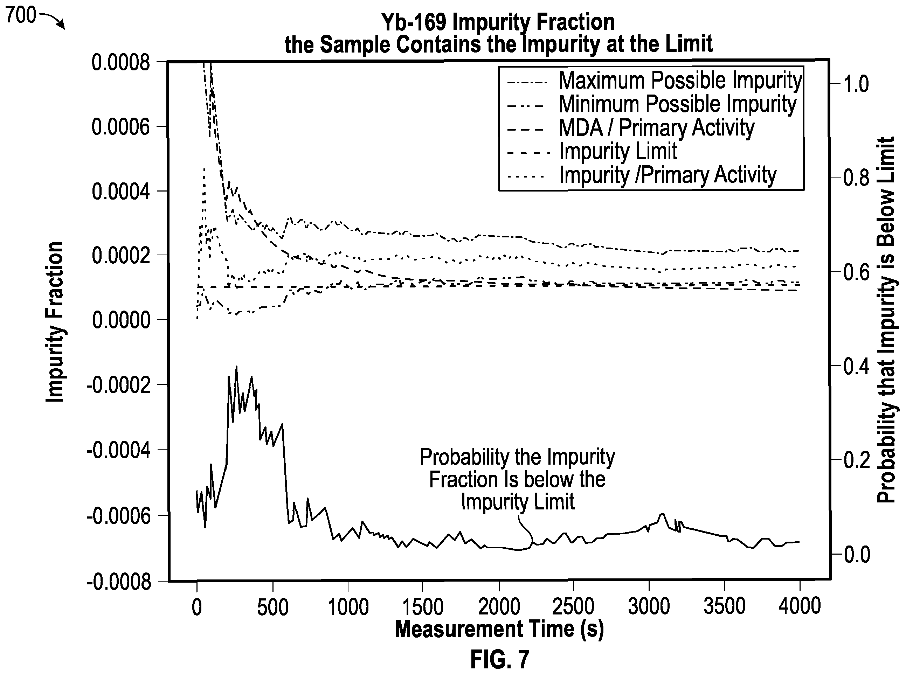

**FIG.** 7 illustrates a plot 700 substantially similar to the plot 500 of FIG. 5, but this time the simulation containing an activity of Yb-169 at the limit. This plot 700 sets forth the maximum possible impurity fraction, the minimum possible impurity fraction, the MDA divided by primary activity, the impurity limit, impurity activity divided by the primary activity, and the probability that the impurity fraction is below the limit for Yb-169 and a sample with the Yb-169 activity at the limit. Once again the probability is plotted using the scale on the right-hand side of the plot.

The maximum possible impurity fraction initially decreases rapidly but then stabilizes and never goes below the limit, which it should not do because the activity of Yb-169 is at the limit. That the minimum probability that the impurity is below the limit reinforces this as well. The relative uncertainty of Yb-169 is high for the entire measurement and, by chance, the calculated activity fraction is higher than the simulated value, but it could just as well have been below. What is important to note is that the maximum possible impurity fraction is always significantly higher than the impurity activity / primary activity, which means that even if the latter ratio is, by chance, below the limit, the maximum possible impurity fraction is still very likely to be above the limit. This is by design because it is required that the results are 95% confident that the true value is below the maximum possible impurity fraction.

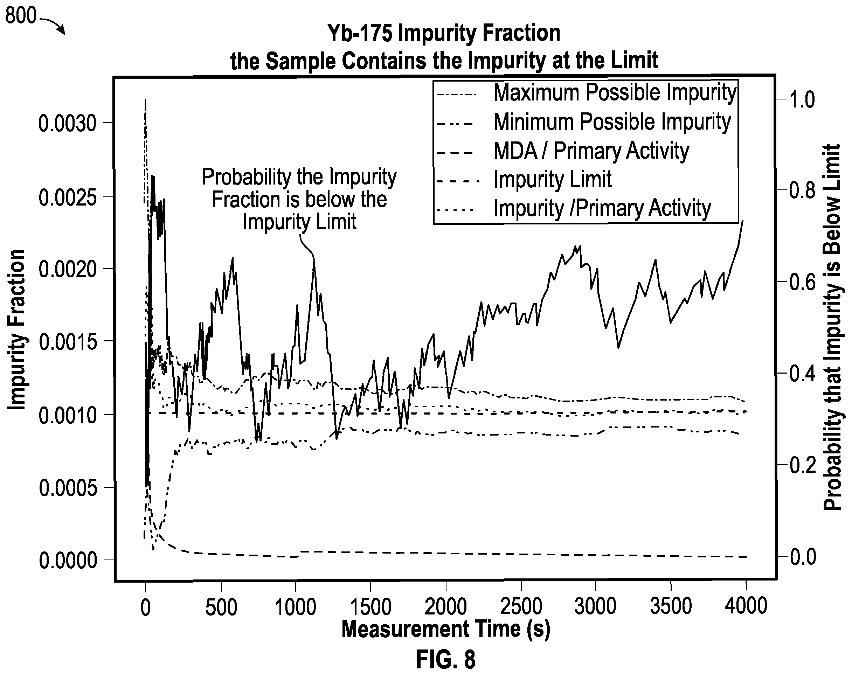

**FIG. 8** illustrates a plot 800 substantially similar to the plot 700 of FIG. 7, but for Yb-175. Specifically, this plot 800 illustrates the maximum possible impurity fraction, the minimum possible impurity fraction, the MDA divided by primary activity, the impurity limit, impurity activity divided by the primary activity, and the probability that the impurity fraction is below the limit for Yb-175 and a sample with the Yb-175 activity at the limit. As before, the probability is plotted using the scale on the right-hand side of the plot.

In the case illustrated in the plot 800 of FIG. 8, the impurity activity / primary activity stays very close to the known value, which is the same as the limit. The maximum possible impurity fraction again never goes below the impurity limit. The probability that the impurity fraction is below the limit fluctuates from about 0.3 to 0.7, highlighting the importance of setting the confidence to a high value or the significance value *α* to a low value to reduce the risk of incorrectly determining that the impurity fraction is below the limit when, in fact, it is close to but above it. The MDA / primary activity goes quickly below the limit because the signal is present. And this shows that the conventional MDA / primary activity gives an uninterpretable result when the signal is present, and it would be necessary to change to some other quantity when the signal is present. As proven by the principles disclosed herein, the maximum possible impurity fraction quantity does not have this limitation and it will always give results that are possible to interpret.

### Conclusions

A new technique and related methods of practical implementation have been developed in accordance with the principles disclosed herein, and prototyped such that it calculates the probability density of the ratio of the activities of two radionuclides (primary and impurity radionuclides) and determines the maximum and minimum values of the ratio to a specific probability. The techniques of the disclosed principles also calculate the probability that the ratio is below a specific limit. A prototype has been implemented in Python and has been tested on Monte Carlo simulations of gamma spectrometry measurements of Lu-177, with the results discussed and detailed above and illustrated in the accompanying figures.

### References

1. Currie, L. Limits for Qualitative Detection and Quantitative Determination. Analytical Chemistry, 1968, 40, 586-593, https://doi.org/10.1021/ac60259a007
2. Šimon, J.; Ftorek, B. Basic Statistical Properties of the Knot Efficiency. Symmetry 2022, 14, 1926. https://doi.org/10.3390/sym14091926
3. ISO11929-1:2019 https://www.iso.org/standard/69579.html
4. ISO11929-2:2019 https://www.iso.org/standard/69580.html

## Claims

1. A method of determining an upper limit with a specified confidence of the ratio of measurements of the activities and their uncertainties of two radionuclides in a sample, the method comprising:
measuring activity of a primary radionuclide and activity of an impurity radionuclide;
calculating an uncertainty of the measured activity of the primary radionuclide and an uncertainty of the measured activity of the impurity radionuclide;
determining a probability density of activity of the primary and impurity radionuclides based on the measured activity and calculated uncertainty of each of the primary and impurity radionuclides;
establishing a probability density of the activity ratio of the impurity radionuclide to the primary radionuclide based on the determined probability density of the primary and impurity radionuclide, respectively;
determining a normalization factor (*N*) to offset when the determined ratio probability density of the activity ratio of the impurity and primary radionuclides is non-zero for negative values;
calculating an upper limit to a specified confidence of the activity ratio of the impurity and primary radionuclides given the measured activities and uncertainties and a-priori knowledge that the activity ratio cannot be negative; and
indicating that the activity ratio of an impurity radionuclide to the primary radionuclide is below a predetermined limit if the calculated upper limit is equal to or below the predetermined limit.

2. The method of claim 1, wherein the measuring activity does not require a test that the measured activity of the impurity radionuclide is present.

3. The method of claim 2, wherein the measuring activity comprises calculating a signal of interest from a background signal subtracted from a gross signal, wherein the background signal is estimated from previous measurements or from information from the current measurement, and wherein uncertainty of the calculated signal of interest is a positive value.

4. The method of claim 1, wherein measuring activity for the impurity radionuclide comprises measuring radiation emitted from the impurity radionuclide at different energies by spectrometry, wherein calculating the upper limit of the activity ratio for the impurity radionuclide to the primary radionuclide comprises calculating multiple upper limits for respective different energies, and wherein the upper limit for the impurity radionuclide comprises the lowest of the multiple upper limits of the activity ratios.

5. The method of claim 4, wherein the minimum possible activity ratio for the impurity radionuclide comprises the lowest of the minimum possible activity ratios for all the activities of the radionuclide.

6. The method of claim 1, wherein determining the normalization factor comprises calculating the integral of the ratio probability density from negative infinity to 0.

7. The method of claim 6, wherein the normalization factor is based on a Cumulative Distribution Function (CDF) responsive to determining the integral of the probability density function of the activity ratio from negative infinity to 0.

8. The method of claim 1, wherein the probability density of the activity ratio is zero for negative activity ratios: ${f}_{Z} \left(z\left|Z\geq 0\right.\right) = \left\{\begin{matrix}1 \left(z\geq 0\right) \\ 0 \left(z<0\right)\end{matrix}\right.$

9. The method of claim 1, wherein the normalization factor for the ratio probability density of the primary and impurity radionuclides comprises: $N = \frac{1}{{\int }_{0}^{z = ∞} {f}_{Z} dz} = \frac{1}{1 - {\int }_{z = - ∞}^{0} {f}_{Z} dz} = \frac{1}{1 - F \left(0\right)}$

10. The method of claim 1, wherein calculating the maximum possible activity ratio comprises calculating zᵤ for which the normalized Probability Density Function (PDF) integral, as normalized by the normalization factor, is equal to 1 - α, where α is the significance level, as defined by: ${F}_{Z} \left({z}_{u}\left|{μ}_{x},{σ}_{{u}_{x}},{μ}_{y},{σ}_{{μ}_{y}},Z\geq 0\right.\right) = \frac{{F}_{Z} \left({z}_{u}\right) - {F}_{Z} \left(0\right)}{1 - {F}_{Z} \left(0\right)} = 1 - α$

11. The method of claim 1, wherein calculating the minimum possible activity ratio comprises calculating *z*ₗ for which the normalized Probability Density Function (PDF) integral, as normalized by the normalization factor, is equal to α, where α is the significance level, as defined by: ${F}_{Z} \left({z}_{l}\left|{μ}_{x},{σ}_{{u}_{x}},{μ}_{y},{σ}_{{μ}_{y}},Z\geq 0\right.\right) = \frac{{F}_{Z} \left({z}_{l}\right) - {F}_{Z} \left(0\right)}{1 - {F}_{Z} \left(0\right)} = α$

12. The method of claim 1, wherein calculating a probability that the true value of the ratio is below a specific limit *zᵥ* is calculated by: $\frac{{F}_{Z} \left({z}_{v}\right) - {F}_{Z} \left(0\right)}{1 - {F}_{Z} \left(0\right)}$

13. The method of claim 1, wherein the measuring activity comprises measuring activity using gamma spectrometry.

14. The method of claim 1, further comprising calculating a lower limit to a specified confidence of the activity ratio of the impurity and primary radionuclides given the measured activities and uncertainties and the a-priori knowledge that the activity ratio cannot be negative.

15. The method of claim 1, wherein determining a probability density of activity of the primary and impurity radionuclides is further based on the activity ratio of the impurity radionuclide as a percentage of the total activity in the sample.

16. The method of claim 1, wherein determining a probability density of activity of the primary and impurity radionuclides is further based on the activity ratio of the impurity radionuclide as a percentage of the activity of the primary radionuclide in the sample.

## Patentansprüche

1. Verfahren zum Bestimmen einer Obergrenze mit einer angegebenen Konfidenz für das Verhältnis von Messungen der Aktivitäten und ihrer Unsicherheiten von zwei Radionukliden in einer Probe, wobei das Verfahren umfasst:
Messen der Aktivität eines primären Radionuklids und der Aktivität eines Verunreinigungsradionuklids;
Berechnen einer Unsicherheit der gemessenen Aktivität des primären Radionuklids und einer Unsicherheit der gemessenen Aktivität des Verunreinigungsradionuklids;
Bestimmen einer Wahrscheinlichkeitsdichte der Aktivität des primären und des Verunreinigungsradionuklids basierend auf der gemessenen Aktivität und der berechneten Unsicherheit jedes des primären und des Verunreinigungsradionuklids;
Ermitteln einer Wahrscheinlichkeitsdichte des Aktivitätsverhältnisses des Verunreinigungsradionuklids zu dem primären Radionuklid basierend auf der jeweils bestimmten Wahrscheinlichkeitsdichte des primären und des Verunreinigungsradionuklids;
Bestimmen eines Normierungsfaktors (*N*) zur Kompensation, wenn die bestimmte Verhältnis-Wahrscheinlichkeitsdichte des Aktivitätsverhältnisses des Verunreinigungsradionuklids und des primären Radionuklids für negative Werte ungleich null ist;
Berechnen einer Obergrenze mit einer angegebenen Konfidenz für das Aktivitätsverhältnis des Verunreinigungsradionuklids und des primären Radionuklids bei gegebenen gemessenen Aktivitäten und Unsicherheiten und bei a-priori-Kenntnis, dass das Aktivitätsverhältnis nicht negativ sein kann; und
Anzeigen, dass das Aktivitätsverhältnis eines Verunreinigungsradionuklids zu dem primären Radionuklid unterhalb eines vorbestimmten Grenzwerts liegt, wenn die berechnete Obergrenze gleich dem vorbestimmten Grenzwert ist oder darunter liegt.

2. Verfahren nach Anspruch 1, wobei das Messen der Aktivität keinen Test erfordert, dass die gemessene Aktivität des Verunreinigungsradionuklids vorhanden ist.

3. Verfahren nach Anspruch 2, wobei das Messen der Aktivität das Berechnen eines interessierenden Signals aus einem von einem Bruttosignal subtrahierten Hintergrundsignal umfasst, wobei das Hintergrundsignal aus früheren Messungen oder aus Informationen aus der aktuellen Messung geschätzt wird, und wobei die Unsicherheit des berechneten interessierenden Signals ein positiver Wert ist.

4. Verfahren nach Anspruch 1, wobei das Messen der Aktivität für das Verunreinigungsradionuklid das Messen von Strahlung umfasst, die von dem Verunreinigungsradionuklid bei unterschiedlichen Energien mittels Spektrometrie emittiert wird, wobei das Berechnen der Obergrenze des Aktivitätsverhältnisses für das Verunreinigungsradionuklid zu dem primären Radionuklid das Berechnen mehrerer Obergrenzen für jeweilige unterschiedliche Energien umfasst, und wobei die Obergrenze für das Verunreinigungsradionuklid die niedrigste der mehreren Obergrenzen der Aktivitätsverhältnisse umfasst.

5. Verfahren nach Anspruch 4, wobei das minimal mögliche Aktivitätsverhältnis für das Verunreinigungsradionuklid das niedrigste der minimal möglichen Aktivitätsverhältnisse für alle Aktivitäten des Radionuklids umfasst.

6. Verfahren nach Anspruch 1, wobei das Bestimmen des Normierungsfaktors das Berechnen des Integrals der Verhältnis-Wahrscheinlichkeitsdichte von minus unendlich bis 0 umfasst.

7. Verfahren nach Anspruch 6, wobei der Normierungsfaktor auf einer kumulativen Verteilungsfunktion (Cumulative Distribution Function, CDF) basiert, die sich aus der Bestimmung des Integrals der Wahrscheinlichkeitsdichtefunktion des Aktivitätsverhältnisses von minus unendlich bis 0 ergibt.

8. Verfahren nach Anspruch 1, wobei die Wahrscheinlichkeitsdichte des Aktivitätsverhältnisses für negative Aktivitätsverhältnisse null ist: ${f}_{Z} \left(z\left|Z\geq 0\right.\right) = \left\{\begin{matrix}1 \left(z\geq 0\right) \\ 0 \left(z<0\right)\end{matrix}\right.$

9. Verfahren nach Anspruch 1, wobei der Normierungsfaktor für die Verhältnis-Wahrscheinlichkeitsdichte des primären und des Verunreinigungsradionuklids umfasst: $N = \frac{1}{{\int }_{0}^{z = ∞} {f}_{Z} dz} = \frac{1}{1 - {\int }_{z = - ∞}^{0} {f}_{Z} dz} = \frac{1}{1 - F \left(0\right)}$

10. Verfahren nach Anspruch 1, wobei das Berechnen des maximal möglichen Aktivitätsverhältnisses das Berechnen von *z*ᵤ umfasst, für das das normierte Integral der Wahrscheinlichkeitsdichtefunktion (Probability Density Function, PDF), wie durch den Normierungsfaktor normiert, gleich 1 - α ist, wobei α das Signifikanzniveau ist, wie definiert durch: ${F}_{Z} \left({z}_{u}\left|{μ}_{x},{σ}_{{u}_{x}},{μ}_{y},{σ}_{{μ}_{y}},Z\geq 0\right.\right) = \frac{{F}_{Z} \left({z}_{u}\right) - {F}_{Z} \left(0\right)}{1 - {F}_{Z} \left(0\right)} = 1 - α$

11. Verfahren nach Anspruch 1, wobei das Berechnen des minimal möglichen Aktivitätsverhältnisses das Berechnen von *z*ₗ umfasst, für das das normierte Integral der Wahrscheinlichkeitsdichtefunktion (Probability Density Function, PDF), wie durch den Normierungsfaktor normiert, gleich α ist, wobei α das Signifikanzniveau ist, wie definiert durch: ${F}_{Z} \left({z}_{l}\left|{μ}_{x},{σ}_{{u}_{x}},{μ}_{y},{σ}_{{μ}_{y}},Z\geq 0\right.\right) = \frac{{F}_{Z} \left({z}_{l}\right) - {F}_{Z} \left(0\right)}{1 - {F}_{Z} \left(0\right)} = α$

12. Verfahren nach Anspruch 1, wobei das Berechnen einer Wahrscheinlichkeit, dass der wahre Wert des Verhältnisses unterhalb eines spezifischen Grenzwerts *z*ᵥ liegt, berechnet wird durch: $\frac{{F}_{Z} \left({z}_{v}\right) - {F}_{Z} \left(0\right)}{1 - {F}_{Z} \left(0\right)}$

13. Verfahren nach Anspruch 1, wobei das Messen der Aktivität das Messen der Aktivität unter Verwendung von Gammaspektrometrie umfasst.

14. Verfahren nach Anspruch 1, ferner umfassend das Berechnen einer Untergrenze mit einer angegebenen Konfidenz für das Aktivitätsverhältnis des Verunreinigungsradionuklids und des primären Radionuklids bei gegebenen gemessenen Aktivitäten und Unsicherheiten und bei der a-priori-Kenntnis, dass das Aktivitätsverhältnis nicht negativ sein kann.

15. Verfahren nach Anspruch 1, wobei das Bestimmen einer Wahrscheinlichkeitsdichte der Aktivität des primären und des Verunreinigungsradionuklids ferner auf dem Aktivitätsverhältnis des Verunreinigungsradionuklids als Prozentsatz der Gesamtaktivität in der Probe basiert.

16. Verfahren nach Anspruch 1, wobei das Bestimmen einer Wahrscheinlichkeitsdichte der Aktivität des primären und des Verunreinigungsradionuklids ferner auf dem Aktivitätsverhältnis des Verunreinigungsradionuklids als Prozentsatz der Aktivität des primären Radionuklids in der Probe basiert.

## Revendications

1. Procédé permettant de déterminer, avec un niveau de confiance spécifié, une limite supérieure du rapport entre les mesures des activités et leurs incertitudes de deux radionucléides présents dans un échantillon, le procédé comprenant :
mesurer l'activité d'un radionucléide primaire et de l'activité d'un radionucléide d'impureté,
calculer une incertitude relative à l'activité mesurée du radionucléide primaire et une incertitude relative à l'activité mesurée du radionucléide d'impureté,
déterminer une densité de probabilité de l'activité des radionucléides primaire et d'impureté sur la base de l'activité mesurée et de l'incertitude calculée pour chacun des radionucléides primaire et d'impureté,
établir une densité de probabilité du rapport d'activité entre le radionucléide impureté et le radionucléide primaire sur la base des densités de probabilité déterminées respectivement pour le radionucléide primaire et le radionucléide impureté,
déterminer un facteur de normalisation (N) à appliquer lorsque la densité de probabilité déterminée du rapport d'activité entre le radionucléide impureté et le radionucléide primaire n'est pas nulle pour les valeurs négatives,
calculer une limite supérieure, avec un niveau de confiance spécifié, du rapport d'activité entre le radionucléide d'impureté et le radionucléide primaire, compte tenu des activités mesurées et des incertitudes, ainsi que de la connaissance a priori selon laquelle le rapport d'activité ne peut être négatif, et
indiquer que le rapport d'activité d'un radionucléide d'impureté par rapport au radionucléide primaire est inférieur à une limite prédéterminée si la limite supérieure calculée est égale ou inférieure à ladite limite prédéterminée.

2. Procédé selon la revendication 1, dans lequel la mesure de l'activité ne nécessite pas de vérifier la présence de l'activité mesurée du radionucléide d'impureté.

3. Procédé selon la revendication 2, dans lequel la mesure de l'activité comprend le calcul d'un signal d'intérêt à partir d'un signal de fond soustrait d'un signal brut, le signal de fond étant estimé à partir de mesures antérieures ou d'informations issues de la mesure en cours, et l'incertitude du signal d'intérêt calculé étant une valeur positive.

4. Procédé selon la revendication 1, dans lequel la mesure de l'activité du radionucléide d'impureté consiste à mesurer, par spectrométrie, le rayonnement émis par le radionucléide d'impureté à différentes énergies, dans lequel le calcul de la limite supérieure du rapport d'activité entre le radionucléide d'impureté et le radionucléide primaire consiste à calculer plusieurs limites supérieures pour chacune des différentes énergies, et dans lequel la limite supérieure pour le radionucléide d'impureté correspond à la plus faible des multiples limites supérieures des rapports d'activité.

5. Procédé selon la revendication 4, dans lequel le rapport d'activité minimal possible pour le radionucléide d'impureté correspond au plus faible des rapports d'activité minimaux possibles pour toutes les activités du radionucléide.

6. Procédé selon la revendication 1, dans lequel la détermination du facteur de normalisation comprend le calcul de l'intégrale de la densité de probabilité du rapport allant de l'infini négatif à 0.

7. Procédé selon la revendication 6, dans lequel le facteur de normalisation est basé sur une fonction de distribution cumulative (CDF) résultant du calcul de l'intégrale de la fonction de densité de probabilité du rapport d'activité allant de l'infini négatif à 0.

8. Procédé selon la revendication 1, dans lequel la densité de probabilité du rapport d'activité est nulle pour les rapports d'activité négatifs : ${f}_{Z} \left(z\left|Z\geq 0\right.\right) = \left\{\begin{matrix}1 \left(z\geq 0\right) \\ 0 \left(z<0\right)\end{matrix}\right.$

9. Procédé selon la revendication 1, dans lequel le facteur de normalisation pour la densité de probabilité du rapport entre les radionucléides primaires et les impuretés comprend : $N = \frac{1}{{\int }_{0}^{z = ∞} {f}_{Z} dz} = \frac{1}{1 - {\int }_{z = - ∞}^{0} {f}_{Z} dz} = \frac{1}{1 - F \left(0\right)}$

10. Procédé selon la revendication 1, dans lequel le calcul du rapport d'activité maximal possible comprend le calcul de zᵤ pour lequel l'intégrale de la fonction de densité de probabilité (PDF) normalisée, normalisée par le facteur de normalisation, est égale à 1 - α, où α est le niveau de signification, tel que défini par : ${F}_{Z} \left({z}_{u}\left|{μ}_{x},{σ}_{{u}_{x}},{μ}_{y},{σ}_{{μ}_{y}},Z\geq 0\right.\right) = \frac{{F}_{Z} \left({z}_{u}\right) - {F}_{Z} \left(0\right)}{1 - {F}_{Z} \left(0\right)} = 1 - α$

11. Procédé selon la revendication 1, dans lequel le calcul du rapport d'activité minimal possible comprend le calcul de z₁ pour lequel l'intégrale de la fonction de densité de probabilité (PDF) normalisée, normalisée par le facteur de normalisation, est égale à α, où α est le niveau de signification, tel que défini par : ${F}_{Z} \left({z}_{l}\left|{μ}_{x},{σ}_{{u}_{x}},{μ}_{y},{σ}_{{μ}_{y}},Z\geq 0\right.\right) = \frac{{F}_{Z} \left({z}_{l}\right) - {F}_{Z} \left(0\right)}{1 - {F}_{Z} \left(0\right)} = α$

12. Procédé selon la revendication 1, dans lequel le calcul d'une probabilité que la valeur réelle du rapport soit inférieure à une limite spécifique zᵥ s'effectue selon la formule suivante : $\frac{{F}_{Z} \left({z}_{v}\right) - {F}_{Z} \left(0\right)}{1 - {F}_{Z} \left(0\right)}$

13. Procédé selon la revendication 1, dans lequel la mesure de l'activité comprend la mesure de l'activité par spectrométrie gamma.

14. Procédé selon la revendication 1, comprenant en outre le calcul d'une limite inférieure, avec un niveau de confiance spécifié, du rapport d'activité entre les radionucléides d'impureté et primaire, compte tenu des activités mesurées et des incertitudes, ainsi que de la connaissance a priori selon laquelle le rapport d'activité ne peut être négatif.

15. Procédé selon la revendication 1, dans lequel la détermination d'une densité de probabilité de l'activité des radionucléides primaires et d'impureté repose en outre sur le rapport d'activité du radionucléide d'impureté exprimé en pourcentage de l'activité totale dans l'échantillon.

16. Procédé selon la revendication 1, dans lequel la détermination d'une densité de probabilité d'activité des radionucléides primaire et d'impureté repose en outre sur le rapport d'activité du radionucléide d'impureté, exprimé en pourcentage de l'activité du radionucléide primaire dans l'échantillon.
